# EUROPÄISCHE PATENTSCHRIFT

(11) **EP 1 463 492 B1**
(45) Veröffentlichungstag und Bekanntmachung des Hinweises auf die Patenterteilung: **26.10.2005**
(21) Anmeldenummer: 02798348.5
(22) Anmeldetag: 19.12.2002
(51) Int. Cl.: A61K 9/70

(54) **HAFT-UND BINDEMITTEL FUR DERMALE ODER TRANSDERMALE THERAPIESYSTEME**
ADHESIVE AND BINDING AGENT FOR DERMAL OR TRANSDERMAL TREATMENT SYSTEMS
AGENT ADHESIF ET LIANT DESTINE A DES SYSTEMES THERAPEUTIQUES DERMIQUES OU TRANSDERMIQUES

(30) Priorität: 09.01.2002 DE 10200578
(43) Veröffentlichungstag der Anmeldung: 06.10.2004
(73) Patentinhaber: Röhm GmbH & Co. KG, 64293 Darmstadt (DE)
(72) Erfinder: ASSMUS, Manfred, 64404 Bickenbach (DE); ROTH, Erna, 64297 Darmstadt (DE); BOIX, Antoni, E-08970 St. Joan Despi (ES); REIG, Isabel, E-08970 St. Joan Despi (ES)
(86) Internationale Anmeldenummer: PCT/EP2002/014556
(87) Internationale Veröffentlichungsnummer: WO 2003/057199

(56) Entgegenhaltungen:
- EP-A- 0 848 950
- EP-A- 0 848 960
- DE-A- 3 843 557
- US-A- 5 290 561
- SANTUS G C ET AL: "TRANSDERMAL ENHANCER PATENT LITERATURE" JOURNAL OF CONTROLLED RELEASE, ELSEVIER SCIENCE PUBLISHERS B.V. AMSTERDAM, NL, Bd. 25, Nr. 1 / 2, 27. Mai 1993 (1993-05-27), Seiten 1-20, XP000361364 ISSN: 0168-3659

## Beschreibung

Die Erfindung betrifft ein Haft- und Bindemittel für dermale oder transdermale Therapiesysteme.

### Stand der Technik

EP-A 315 218 beschreibt pharmazeutische Zusammensetzungen zur transdermalen systemischen Verabreichung von pharmakologischen Wirkstoffen, dadurch gekennzeichnet, daß sich die pharmakologischen Wirkstoffe in einem Reservoir befinden, das ein Polyacrylatpolymer mit kationischen Eigenschaften enthält. Weichmacher und können in Mengen von 3 bis 33 Gew.-% enthalten sein. Penetrationsförderer können in Mengen 10 bis 100 Gew.-% enthalten sein. Die pharmazeutische Zusammensetzung kann zusätzlich mit einer Klebeschicht versehen werden, um eine gute Haftung auf der Haut zu erreichen.

EP-A 617 972 beschreibt schichtförmige dermale therapeutische Systeme mit verzögerter Wirkstoffabgabe, die aus Mischungen von Poly(meth)acrylaten bestehen und aus einer Schmelze hergestellt werden. Dabei enthält eine Poly(meth)acrylat-Komponente funktionelle Gruppen, während eine weitere Poly(meth)acrylat-Komponente keine oder nur unerhebliche Mengen an funktionellen Gruppen enthält und im wesentlichen das Fließverhalten der polymeren Klebeschicht reguliert. Penetrationsförderer werden als mögliche Zusatzstoffe genannt.

EP-A 848 950 und EP-A 848 960 beschreiben Haft- und Bindemittel für pharmazeutische Zwecke, die sich durch eine hohe Hydrophilie bzw. hohe Wasserdampfdurchlässigkeit auszeichnen und dabei gleichzeitig eine hohe Klebkraft bei geringem Kaltfluß aufweisen. Die Haft- und Bindemittel eignen sich deshalb hervorragend als Hauthaftkleber oder transdermale Therapiesysteme.

EP-A 0 848 960 beschreibt ein Haft- und Bindemittel für dermale oder transdermale Therapiesysteme bestehend aus (a1) 55 - 99,9 Gew.-% eines (Meth)acrylatcopolymer aus strukturellen und funktionellen Monomeren, wobei die funktionellen Monomeren tertiäre oder quaternäre Aminogruppen aufweisen, (a2) 0,1 - 45 Gew.-% eines säuregruppenhaltigen Acrylat- oder (Meth)acrylat Polymeren oder Copolymeren und (b) 25 - 80 Gew.-%, bezogen auf die Summe von (a1) und (a2), eines Weichmachers.

EP-A 848 950 beschreibt ein Haft- und Bindemittel für dermale oder transdermale Therapiesysteme bestehend aus (a) 85 - 99 Gew.-% eines (Meth)acrylatcopolymers aus strukturellen und funktionellen Monomeren, wobei die funktionellen Monomeren tertiäre oder quaternäre Aminogruppen aufweisen, (b) 15 - 0,1 Gew.-% einer organischen Di- oder Tricarbonsäure sowie (c) 40 - 70 Gew.-%, bezogen auf die Summe von (a) und (b), eines Weichmachers.

DE 40 20 144 C2 beschreibt eine Vorrichtung zur topischen und systemischen Verabreichung von Wirkstoffen. Es handelt sich um Mischungen von Polyacrylatkleber mit einem Filmbildner, z. B einem neutralen Copolymer aus Methylmethacrylat und Butylmethacrylat (PLASTOID® B), die weichmachende Hilfsstoffe wie Solubilisatoren oder Penetrationsbeschleuniger, z. B. Fettalkohole wie Dodecanol, in Mengen von z. B. 20 Gew.-% enthalten können.

### Aufgabe und Lösung

Ein grundsätzliches Problem bei Haft- und Bindemittel für dermale oder transdermale Therapiesysteme ist es, die Komponenten so abzustimmen, daß gleichzeitig ein Reihe von Parametern erfüllt werden. Geeignete Bestimmungsmethoden für diese im folgenden genannten Parameter sind dem Fachmann auf dem Gebiet der dermalen oder transdermalen Therapiesysteme geläufig (siehe dazu z. B. auch die Ausführungen im Punkt "Beispiele).

Zu nennen ist zunächst eine ausreichende Klebkraft, die jedoch nur so hoch sein soll, daß es bei Ablösen der Klebschicht zu einem Adhaesionsbruch, nicht jedoch zu einem Kohäsionsbruch kommt.

Weiterhin sind Mindestanforderungen an die Eigenschaften Tack und Kaltfluß zu erfüllen. Der Tack soll generell mindestens den Wert 1 erreichen, der Kaltfluß nicht mehr als 1 mm/24 Stunden betragen.

Schließlich soll ein enthaltener pharmazeutischen Wirkstoff gut in die Haut eindringen. Man strebt Penetrationswerte von mindestens 1 µg Wirkstoff/cm² an.

Gerade letzteres Ziel ist nur schwer erreichbar, da dies den Zusatz vergleichsweise hoher Mengenanteile an sich bekannter, penetrationsfördernder Substanzen erfordert. Die erforderlichen relativ hohen Mengenanteile wirken sich neben anderen erforderlichen Komponenten wie Weichmachern meist negativ auf das Gesamtsystem aus. Vermindert man hingegen zugunsten der penetrationsfördernden Substanz den Gehalt anderer Komponenten, z. B. den des Weichmachers, erreicht man zwar die gewünschte Penetrationsrate, die übrigen Eigenschaften, insbesondere die Klebkraft, liegen dann in der Regel jedoch nicht mehr im angestrebten Bereich.

Es wurde daher als Aufgabe gesehen, ein Haft- und Bindemittel für dermale oder transdermale Therapiesysteme bereitzustellen, daß möglichst alle eingangs genannten Kriterien gleichzeitig erfüllt. Weiterhin sollte angestrebt werden, den Gehalt an nicht penetrationsfördernenden weichmachenden Substanzen möglichst herabzusetzen oder ganz darauf zu verzichten, damit der Gehalt der übrigen Inhaltstoff- und Zusatzstoffe gegenüber dem Gehalt am eigentlichen matrixbildenden System nicht zu hoch wird, so daß z. B. eine kontrollierte Wirkstoffabgabe nicht beeinträchtigt wird.

Überraschenderweise wurde gefunden, daß die Aufgabe gelöst wird durch ein Haft- und Bindemittel für dermale oder transdermale Therapiesysteme enthaltend
(a) ein (Meth)acrylat-Copolymer aus radikalisch polymerisierten C₁- bis C₄-Alkylestern der Acryl- oder der Methacrylsäure und (Meth)acrylatMonomeren mit einer **tertiären oder quaternären Aminogruppe** im Alkylrest, enthaltend
(b) 0,1 - 45 Gew.-% bezogen auf (a) einer organischen Di- oder Tricarbonsäure oder eines säuregruppenhaltigen Acrylat- oder (Meth)acrylat Polymeren oder Copolymeren sowie
(c) eine in Bezug auf die Haut penetrationsfördernde Substanz
(d) gegebenenfalls einen pharmazeutischen Wirkstoff, einen Weichmacher und/oder einen oder mehrere pharmazeutisch übliche Zuschlagstoffe
dadurch gekennzeichnet, daß
die penetrationsfördernde Substanz (c) ein Alkohol mit 10 bis 12 Kohlenstoffatomen ist **und höchstens 20 Gew.-% eines Weichmachers bezogen auf die Komponente (a) enthalten sind.**

### Ausführung der Erfindung

### Komponente (a)

Die Komponente (a) ist ein (Meth)acrylat-Copolymer aus radikalisch polymerisierten C₁- bis C₄-Alkylestern der Acryl- oder der Methacrylsäure und (Meth)acrylat-Monomeren mit einer kationischen Ammoniumgruppe im Alkylrest. Unter diese Definition fallen Copolymere, die u. a. unter den Produktnamen EUDRAGIT® E, EUDRAGIT® RS oder EUDRAGIT® RL seit langem als Arzneimittelüberzüge bekannt sind.

Bevorzugt beträgt der Gehalt der (Meth)acrylat-Monomeren mit einer kationischen Ammoniumgruppe im Alkylrest mindestens 2, bevorzugt mindestens 4 Gew.-% bezogen auf das Copolymer.

C₁- bis C₄- Alkylester der Acryl- oder Methacrylsäure sind insbesondere Methyacrylat, Ethyacrylat, Butylacrylat, Butylmethacrylat und Methylmethacrylat.

(Meth)acrylat-Monomeren mit einer kationischen Ammoniumgruppe im Alkylrest sind insbesondere (Meth)acrylat-Monomere mit tertiären oder quaternären Amino- bzw. Ammoniumgruppen im Alkylrest.

Der Gehalt der funktionellen Monomere mit tertiären Ammoniumgruppen kann vorteilhafterweise 30 bis 70 Gew.-%, bevorzugt 40 bis 60 Gew.-% betragen.

Geeignete Monomere mit tertiären Ammoniumgruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat. Besonders bevorzugt ist Dimethylaminoethylmethacrylat.

Ein der Komponente (a) entsprechendes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein.

Ein der Komponente (a) entsprechendes (Meth)acrylatcopolymer mit tertiären Aminogruppen kann z. B. aus 25 Gew.-% Methylmethacrylat, 25 Gew.-% Butylmethacrylat und 50 Gew.-% Dimethylaminoethylmethacrylat aufgebaut sein (EUDRAGIT® E 100).

Der Gehalt der funktionellen Monomere mit quaternären Ammoniumgruppen kann bevorzugt 2 bis 15, besonders bevorzugt 4 bis 12 Gew.-% betragen. Als Monomer mit funktionellen quaternären Ammoniumgruppen wird 2-Trimethylammoniumethlymethacrylat-Chlorid besonders bevorzugt.

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungverfahren ist die Substanzpolymeriation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise in Form eines feinen Pulvers erhalten werden, was bei der Subtanzpolymerisation durch Mahlen, bei Lösungs- und Fällungspolymerisation z. B. durch Sprühtrocknung erreichbar ist.

Ein entsprechendes Copolymer, kann z. B. aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein.

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylat-Monomere sind handelsüblich und werden seit langem für retardierende Überzüge verwendet.

Ein der Komponente (a) entsprechendes (Meth)acrylatcopolymer mit quarternären Aminogruppen , kann z. B. aus 60 Gew.-% Methylmethacrylat, 30 Gew.-% Ethylacrylat und 10 Gew.-% 2-Trimethylammoniumethlymethacrylat-Chlorid aufgebaut sein (EUDRAGIT® RL 100).

Ein weiteres bevorzugtes der Komponente (a) entsprechendes (Meth)acrylatcopolymer mit mit quarternären Aminogruppen kann z. B. aus 65 Gew.-% Methylmethacrylat, 30 Gew.-% Ethylacrylat und 5 Gew.-% 2-Trimethylammoniumethlymethacrylat-Chlorid aufgebaut sein (EUDRAGIT® RS 100).

Die Copolymere (a) werden in an sich bekannter Weise durch radikalische Substanz-, Lösungs-, Perl- oder Emulsionspolymerisation erhalten. Sie können als extrudiertes Granulat, gemahlenes Pulver, Lösung oder Dispersion vorliegen.

### Komponente (b)

Die Komponente (b) fungiert als Gegenion zur kationischen Komponente (a). Die Komponente (b) kann dabei so eingestellt werden, daß eine teilweise oder vollständige Neutralisation bewirkt wird. Die kationischen Reste des kationischen (Meth)acrylat-Copolymers (a) werden mittels der Komponente (b) bevorzugt zu 2 bis 100 %, besonders bevorzugt zu 5 bis 60 neutralisiert.

Die Komponente (b) besteht, bezogen auf die Komponente (a) aus 0,1 bis 45, bevorzugt 1 bis 30, besonders bevorzugt 5 bis 25 Gew.-% bezogen auf (a) einer organischen Di- oder Tricarbonsäure oder eines säuregruppenhaltigen Acrylat- oder (Meth)acrylat Polymeren oder Copolymeren. Bevorzugt ist wird eine organischen Di- oder Tricarbonsäure eingesetzt, da deren Mengenanteil meist auf 0,1 bis 18, bevorzugt 5 bis 15 Gew.-% beschränkt werden.

Geeignet sind organische Di- Tricarbonsäuren, bevorzugt Bernsteinsäure (Succinat), Fumarsäure oder Citronensäure.

Eine weitere geeignete Komponente (b) sind säuregruppenhaltige Acrylat- oder (Meth)acrylat Polymere oder Copolymere.

Geeignet ist z. B. Polyacrylsäure (®Carbopol).

Bevorzugt sind jedoch Copolymere aus strukturellen und funktionellen (Meth)acrylat-Monomeren. Strukturelle Acryl- oder Methacrylat-Monomere sind z. B. C₁- bis C₄- Alkylester der Acryl- oder Methacrylsäure. Bevorzugt sind Methyacrylat, Ethyacrylat, Butylacrylat und Methylmethacrylat. Als Monomer mit funktionellen Säuregruppen wird Methacrylsäure besonders bevorzugt.

Ein der Komponente (b) entsprechendes Copolymer kann z. B. aus 30 - 70 Gew.-% Ethylacrylat oder Methylmethacrylat und 70 - 30 Gew.-% Methacrylsäure aufgebaut sein.

Wesentlich für die vorliegende Erfindung ist, daß die Komponenten (a) und (b) in den angegebenen Verhältnissen vorliegen. Beträgt der Anteil des säuregruppenhaltigen Copolymers (b) weniger als 0,1 Gew.-%, so ist der Kaltfluss in der Regel zu hoch. Liegt der Anteil über 45 Gew.-%, so hat dies den Nachteil, daß die Verarbeitbarkeit beeinträchtigt ist.

### Komponente (c)

Die Komponente (c) ist eine penetrationsfördernde Substanz, die ein Alkohol mit 10 bis 12 Kohlenstoffatomen ist. Die Komponente (c) kann z. B. Decanol, bzw. n-Decanol, Undecanol (1-Undecanol oder 2-Undecanol) oder Dodecanol bzw. n-Dodecanol ist.

### Pharmazeutische Wirkstoffe

Wichtige Beispiele für geeigente pharmazeutischer Wirkstoffe (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
Broncholytika/Antiasthmatika, Cholinergika, Corticoide (Interna),
Dermatika, Diuretika, Enzyminhibitoren, Enzympräparate und Transportproteine,
Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.

### Beispiele für Wirkstoffe sind:

Die Erfindung eignet sich sich insbesondere für die Bereitstellung von Arzneiformen enthaltend die unten stehenden Wirkstoffe.

### Therapeutische Kategorien:

Analgetika, Antirheumatika, Antiallergika, Antiarrhythmika, Betarezeptorenblocker, Calciumkanalblocker, Hemmstoffe des Renin-Angiotensin-Systems, Broncholytika/Antiasthmatika, Cholinergika, Diuretika Durchblutungsfördernde Mittel, Gichtmittel, Grippemittel, Koronarmittel, Lipidsenker Magen-Darmmittel, Psychopharmaka, Thrombozytenaggregationshemmer, Urologika, Venetherapeutika, Vitamine und Mineralien

### Wirkstoffe

Morphin und/oder dessen Derivate,Tramadol, Acetylsalicylsäure, Diclofenac, Indometacin, Lonazolac, Ibuprofen, Ketoprofen, Propyphenazon, Naproxen, Paracetamol, Flurbiprofen, Dimetinden, Chinidin, Metoprolol, Propranolol, Oxprenolol, Pindolol, Atenolol, Metoprolol, Disopyramid, Verapamil, Diltiazem, Gallopamil, Nifedipin, Nicardipin, Nisoldipin, Nimodipin, Amlodipin, Theophyllin, Salbutamol, Sildenafil, Terbutalin, Ambroxol, Aminophyllin, Cholintheophyllinat, Pyridostigmin, Piretanid, Furosemid, Pentoxifyllin, Naftidrofuryl, Buflomedil, Xantinolnicotinat, Bencyclan, Allopurinol, Norephedrin, Clorphenamin Isosorbidmononitrat, Isosorbiddinitrat, Glyceroltrinitrat, Molsidomin, Bezafibrat, Fenofibrat, Gemfibrozil, Cerivastatin, Pravastatin, Fluvastatin, Lovastatin, Atorvastatin, Simvastatin, Xantinol, Metoclopramid, Amitriptylin, Dibenzepin, Venlafaxin, Thioridazin, Oxazepam, Lithium, Nitrofurantoin, pflanzliche Trockenextrakt, Ascorbinsäure und Kalium und/oder deren pharmazeutisch verwendete Salze.

Wichtige Wirkstoffe für transdermale Therapiesysteme sind insbesondere Nicotin, Glyceroltrinitrat, Scopolamin, Clonidin, Fentanyl, Östradiol, Testosteron, Oxibutynin, Diclophenac, Desoxyribonukleinsäuren z. B. für Vaccinen, Ibuprofen, Ketoprofen, Diltiazem, Propranolol, Albuterol, Alprazolam, Amethocaine, Atenolol, Benzoporphyrin, Buprenorphine, Calcitonin, Dithranol, Diphencypron, hautpenetrierende bzw. durch die Haut resorbierbare Peptide, Eptazocine, Ethinylöstradiol, Methrotrexat oder Naloxon.

Übliche Mengenanteile für pharmazeutische Wirkstoffe sind 10 bis 100 Gew.-% beogen auf die Komponente (a).

### Weichmacher

Der Weichmacherzusatz erlaubt die Anpassung physikalischer Eigenschaften an die Erfordernisse der einzelnen Arzneiformen, sodaß bei Raum- bzw. Körpertemperatur ausreichende Haftkräfte erreicht werden.
Weichmacher in den angegebenen Verhältnissen können die Schmelzviskosität der eingesetzten Polymere im flüssigen Zustand erniedrigen. Bei Raumtemperatur sind erweichende Effekte zu erkennen.
Einflüsse auf das Freigabeverhalten eingebetteter Wirkstoffe sind möglich.
Ein Weichmacher, soll zu höchstens 20, bezogen auf die Komponente (a) vorhanden sein. Besonders bevorzugt ist kein Weichmacher enthalten.

Als Weichmacher geeignete Stoffe haben in der Regel ein Molekulargewicht zwischen 100 und 20 000 und enthalten eine oder mehrere hydrophile Gruppen im Molekül, z. B. Hydroxyl-, Ester- oder Aminogruppen. Geeignet sind Citrate, Phthalate, Sebacate, Rizinusöl. Beispiele geeigneter Weichmacher sind Citronensäurealkylester, Glycerinester, Phtalsäurealkylester, Sebacinsäurealkylester, Sucroseester, Sorbitanester, Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen **bevorzugt** zwischen **1** und **höchstens 20,** bevorzugt 2 bis 10 Gew.-%, bezogen auf das (Meth)acrylat-Copolymere.

### Pharmazeutisch übliche Zuschlagstoffe

Pharmazeutisch übliche Zuschlagstoffe können sein: Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Weitere pharmazeutisch übliche Hilfsstoffe können in Mengen von 0,001 Gew-% bis 100 Gew.-%, bevorzugt 0,1 bis 50 Gew.-% bezogen auf das Copolymere vorliegen. Beispiele für Trockenstellmittel sind: Aluminiumoxid, Magnesiumoxid, Kaolin, Talkum, Kieselsäure (Aerosile), Bariumsulfat, Ruß und Cellulose. Beispiele für Trennmittel (Formtrennmittel) sind: Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc..

Variationen der Zusammensetzung ermöglichen es gegebenenfalls unerwünschte Effekte von arzneiformbedingten Zusätzen auszugleichen. Die erfindungsgemäßen Haft- und Bindemittel können optional weitere Zusätze in geringen Mengen enthalten, wenn es die spezielle Formulierung erfordert: Neutrale Polymere, Tackifyer, Stabilisatoren, Farbstoffe, Antioxidantien, Netzmittel, Porenbildner, Feuchthaltemittel, komplexierende Mittel u.a..

### Herstellungsverfahren:

Die Herstellung des transdermalen Therapiesystems hängt von der eingesetzten Form des Polymeren ab: Festsubstanzen können direkt eingesetzt werden durch Mischen mit den Zusatzstoffen in geeigneten Mischern, Knetern oder Extrudern, die heizbare und ggf. evakuierbar sind. Der Extruder ist ein - oder bevorzugt doppelschneckig, um geeignete Misch- und Transporteigenschaften zu erreichen.

Die Verarbeitungstemperatur richtet sich nach den Schmelzeigenschaften der Materialien und liegt vorzugsweise zwischen 20°C und 200°C. Einschränkende Faktoren sind die thermische Stabilität der Einsatzstoffe. Feste Zuschlagstoffe können vor der Extrusion mit den Polymer gemischt werden. Flüssige Zuschlagstoffe werden auf etwa der halben Extrusionsstrecke der Schmelze zugesetzt und bewirken eine Viskositätserniedrigung und Temperaturabsenkung.

Polymerlösungen oder Dispersionen werden mit den Zusatzstoffen versetzt, so daß diese sich auflösen oder suspendiert werden. Aus diesen Lösungen, Dispersionen oder Suspensionen erhält man das Bindemittel durch Trocknen zu dünnen Filmschichten.

### Verarbeitung:

Beschichtung, Granulation, Umhüllung oder Einbettung erfolgen mittels organischer Lösung oder wäßriger Dispersion von geeigneten Hilfsstoffen.
Die Verwendung von Schmelzen beschränkt sich auf Substanzen mit definierten Schmelzpunkten im Bereich der Verarbeitungstemperaturen. Üblicherweise benötigt man niedrige Schmelzviskositäten für die Verarbeitung.

In einer Verfahrensvariante wird das erfindungsgemäße, feste Haft- und Bindemittel mit den Pulvern gemischt und mit einem geeigneten Lösungsmitten gemischt oder gemeinsam aufgeschmolzen.

Bevorzugt aus Lösung bzw. Suspension oder direkt aus der Schmelze erhält man durch Ausstreichen auf flächige Träger z.B. Folien, Gewebe oder Vliese, nach Trocknung oder Abkühlung Haftschichten, die das System auf der Haut fixieren und wegen der Hydrophilie besonders gut verträglich sind. Die Beschichtung erfolgt im Labor diskontinuierlich mittels einer Rakel und im Technikum und Produktion kontinuierlich mittels Rollrakel oder Walzenauftrag. Direkt nach der Beschichtung fügt man eine schwach haftende, oft silikonisierte Deckfolie hinzu, die vor der Anwendung entfernt wird.

Die erhaltenen Agglomerate oder Haftschichten können für die Anwendung zu Arzneiformen weiterverarbeitet werden. Dabei ist es möglich Arzneistoffe schon während der Herstellung des Haft- und Bindemittels einzuarbeiten. Diese Wirkstoffe sind dann in partikulärer oder gelöster Form fixiert. Eine Beeinflussung der Wirkstoffabgabe durch das Haft- und Bindemittel ist möglich und kann für die Formulierung von Arzneiformen ausgenutzt werden.

### Arzneiformen

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Erfindungsgemäß können alle Wirkstoffe eingesetzt werden, die die gewünschte therapeutische Wirkung im Sinne der obigen Definition erfüllen.

Arzneiformen lassen sich aus den erfindungsgemäß hergestellten Zwischenstufen durch übliche Verabeitungstechniken herstellen.

Mit dem Haft- und Bindemittel bestrichene Träger liegen in der Regel auf Rollen vor, geschützt durch Deckfolien (release liner). Aus diesen Bahnen werden einzelne Pflaster der erforderlichen Größe geschnitten oder gestanzt und einzeln verpackt.

Das Beschichten flächiger Träger mit polymerhaltigen Flüssigkeiten ist z. B. in Mass, J. und Schmidt, H. : Coating Technology for Transdermal Drug Delivery Systems, Medical Device Technology, Ausgabe 3/41990, S. 46 - 50, beschrieben.

Für die Applikation relevante Eigenschaften, geforderte Tests und Spezifikationen sind in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- Heilmann, K. : Therapeutische Systeme, Ferdinand Euler Verlag, Stuttgart, S. 52 -57.
- Brandau, R. und Lippold, B. H. (1982) : Dermal and Transdermal Absorption. Wissenschaftliche Verlagsgesellschaft mbH, Stuttgart, S. 171 - 200.

### BEISPIELE

### Ausführung der Beispiele:

EUDRAGIT® E100: Copolymer. aus 25 Gew.-% Methylmethacrylat, 25 Gew.-% Butylmethacrylat und 50 Gew.-% Dimethylaminoethylmethacrylat.
PLASTOID® B: Copolymer aus 50 Gew.-% Methylmethacrylat und 50 Gew.-% Butylmethacrylat.
Durotak® 280 2516: Polyacrylatkleber (Bestandteile gemäß rote Liste: Acrylat/Vinylacetat/Methacrylat-Copolymer)

### Beispiel 1 (Vergleichsbeispiel)

100 g EUDRAGIT® E100 (Copolymer. aus 25 Gew.-% Methylmethacrylat, 25 Gew.-% Butylmethacrylat und 50 Gew.-% Dimethylaminoethylmethacrylat) werden in einer Mischung aus 53,6 g Aceton, 29,8 g Ethanol und 6,0 g Isopropanol unter rühren gelöst.
In diese Lösung werden 40 g Dibutylsebacat, 12 g Bernsteinsäure und 16 g Alprazolam eingearbeitet. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Beispiel 2

100 g EUDRAGIT® E100 (Copolymer. aus 25 Gew.-% Methylmethacrylat, 25 Gew.-% Butylmethacrylat und 50 Gew.-% Dimethylaminoethylmethacrylat) werden in einer Mischung aus 53,6 g Aceton, 29,8 g Ethanol und 6,0 g Isopropanol unter rühren gelöst.
In diese Lösung werden 40 g Dodecanol, 12 g Bernsteinsäure und 16 g Alprazolam eingearbeitet. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Beispiel 3

100 g EUDRAGIT® E100 (Copolymer. aus 25 Gew.-% Methylmethacrylat, 25 Gew.-% Butylmethacrylat und 50 Gew.-% Dimethylaminoethylmethacrylat) werden in einer Mischung aus 53,6 g Aceton, 29,8 g Ethanol und 6,0 g Isopropanol unter rühren gelöst.
In diese Lösung werden 40 g Decanol, 9 g Bernsteinsäure und 16 g Alprazolam eingearbeitet. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Beispiel 4

100 g EUDRAGIT® E100 (Copolymer. aus 25 Gew.-% Methylmethacrylat, 25 Gew.-% Butylmethacrylat und 50 Gew.-% Dimethylaminoethylmethacrylat) werden in einer Mischung aus 53,6 g Aceton, 29,8 g Ethanol und 6,0 g Isopropanol unter rühren gelöst.
In diese Lösung werden 20 g Decanol, 20 g Dibutylsebacat, 12 g Bernsteinsäure und 16 g Alprazolam eingearbeitet. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Beispiel 5 (Vergleichsbeispiel)

100 g EUDRAGIT® E100 (Copolymer. aus 25 Gew.-% Methylmethacrylat, 25 Gew.-% Butylmethacrylat und 50 Gew.-% Dimethylaminoethylmethacrylat) werden in einer Mischung aus 53,6 g Aceton, 29,8 g Ethanol und 6,0 g Isopropanol unter rühren gelöst.
In diese Lösung werden 50 g Myristinalkohol, 9 g Bernsteinsäure und 16 g Alprazolam eingearbeitet. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Beispiel 6 (Vergleichsbeispiel)

100 g PLASTOID® B werden in einer Mischung aus 53,6 g Aceton, 29,8 g Ethanol und 6,0 g Isopropanol unter rühren gelöst.
In diese Lösung werden 40 g Dodecanol, Alprazolam eingearbeitet. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Beispiel 7 (Vergleichsbeispiel)

100 g PLASTOID® B werden in einer Mischung aus 53,6 g Aceton, 29,8 g Ethanol und 6,0 g Isopropanol unter rühren gelöst.
In diese Lösung werden 50 g Decanol, Alprazolam eingearbeitet. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Beispiel 8 (Vergleichsbeispiel)

100 g Durotak® 280 2515 werden mit 30 g Decanol gemischt. Auf einer 50µm dicken Aluminiumfolie wird eine 160 µm dicke Schicht des Klebers bei 60°C 10 Minuten getrocknet. Man erhält eine klare ca. 100 µm dicke Schicht.

### Meßmethoden:

### Bestimmung des Tacks:

Ein Probantenkollektiv bring ein ca. 16 cm² großes Pflaster auf die Haut auf und beurteilt nach folgender Klassifizierung:
0 = Das System haftet nicht auf der Haut, selbst nach 1 Minute leichten Anpressens.
1 = Das System haftet auf der Haut mit maximal 1 Minute leichtem Anpressen.
2 = Das System haftet auf der Haut ohne zusätzliches Anpressen.

### Bestimmung des Kaltflusses:

Ein Probantenkollektiv bring ein ca. 16 cm² großes Pflaster auf die Haut auf und trägt dieses unter gleichbleibenden Bedingungen über 24 Stunden. Danach wird das Wandern bzw. Verrutschen des Pflasters in mm gemessen.

### Bestimmung des Bruchverhaltens (Adhaesions- bzw. Kohaesionsbruch) und der Klebrigkeit:

Die Bestimmung erfolgt durch Abziehen eines 50 mm breiten beschichteten Streifens, bevorzugt aus Aluminium, von einer VA-Stahlplatte, bei gleichzeitiger Messung der dafür benötigten Kraft.

### Bestimmung der Penetration:

Die Bestimmung erfolgte mit einer modifizierten Franzzelle und Humanhaut.

**Tabelle:**

| **Ergebnisse der Beispiele 1 bis 8: (Beispiele 2 bis 4 = erfindungsgemäß; Beispiele 1, 5 bis 7, 8 = Vergleichsbeispiele)** | | | | | | | | |
|---|---|---|---|---|---|---|---|---|
| **Beispiel Nr.** | **1** | **2** | **3** | **4** | **5** | **6** | **7** | **8** |
| Eudragit E100 | 100 | 100 | 100 | 100 | 100 | | | |
| Plastoid B | | | | | | 100 | 100 | |
| Durotak 280 2516 | | | | | - | | | 100 |
| Succinat | 12 | 12 | 9 | 12 | 9 | | | |
| Dibutylsebacat | 40 | - | - | 20 | - | - | - | - |
| Decanol | | | 40 | 20 | | | 50 | 50 |
| Dodecanol | - | 40 | | | | 40 | | |
| Myristinalkohol | | | | | 50 | | | |
| Tack (Sollwert = min.1) | 1 | 2 | 2 | 2 | 0 | 0 | 0 | 2 |
| Kaltfluß [mm/24 h] (Sollwert = max.1) | 0 | 0,08 | 0,07 | 0,05 | - | - | - | 5 |
| Adhaesionsbruch (Soll = +) | + | + | + | + | + | + | + | - |
| Klebkraft [cm/min] (Sollwert = min. 1) | 2,3 | 5,0 | 4,4 | 3,0 | 0 | 0 | 0 | n.b. |
| Penetration [µg/cm²] (Sollwert = min. 1) | 0,5 | 1,9 | 2,8 | 2,4 | n.b. | n.b. | n.b. | n.b. |
| n.b.= nicht bestimmt | | | | | | | | |

## Patentansprüche

1. Haft- und Bindemittel für dermale oder transdermale Therapiesysteme enthaltend
(a) ein (Meth)acrylat-Copolymer aus radikalisch polymerisierten C₁- bis C₄-Alkylestern der Acryl- oder der Methacrylsäure und (Meth)acrylatMonomeren mit einer **tertiären oder quaternären Aminogruppe** im Alkylrest, enthaltend
(b) 0,1 - 45 Gew.-% bezogen auf (a) einer organischen Di- oder Tricarbonsäure oder eines säuregruppenhaltigen Acrylat- oder (Meth)acrylat Polymeren oder Copolymeren sowie
(c) eine in Bezug auf die Haut penetrationsfördernde Substanz
(d) gegebenenfalls einen pharmazeutischen Wirkstoff, einen Weichmacher und/oder einen oder mehrere pharmazeutisch übliche Zuschlagstoffe
**dadurch gekennzeichnet, daß**
die penetrationsfördernde Substanz (c) ein Alkohol mit 10 bis 12 Kohlenstoffatomen ist **und höchstens 20 Gew.-% eines Weichmachers bezogen auf die Komponente (a) enthalten sind.**

2. Haft- und Bindemittel nach Anspruch 1, **dadurch gekennzeichnet, daß** die penetrationsfördernde Substanz (c) Decanol, Undecanol oder Dodecanol ist.

3. Haft- und Bindemittel nach Anspruch 1 oder 2, **dadurch gekennzeichnet, daß** sich das kationische (Meth)acrylat-Copolymer (a) aus 30 bis 80 Gew.-% radikalisch polymerisierten C₁- bis C₄-Alkylestern der Acryl- oder der Methacrylsäure und 70 bis 20 Gew.-% (Meth)acrylat-Monomeren mit einer tertiären b im Alkylrest zusammensetzt.

4. Haft- und Bindemittel nach einem oder mehreren der Ansprüche 1 bis 3, **dadurch gekennzeichnet, daß** sich das kationische (Meth)acrylat-Copolymer (a) aus 85 bis 98 Gew.-% radikalisch polymerisierten C1- bis C4-Alkylestern der Acryl- oder der Methacrylsäure und 15 bis 2 Gew.-% (Meth)acrylatMonomeren mit einer quaternären **Aminogruppe** im Alkylrest zusammensetzt.

5. Haft- und Bindemittel nach einem oder mehreren der Ansprüche 1 bis 4, **dadurch gekennzeichnet, daß** als organische Di- Tricarbonsäuren (b) Bernsteinsäure (Succinat), Fumarsäure oder Citronensäure enthalten sind.

6. Haft- und Bindemittel nach einem oder mehreren der Ansprüche 1 bis 4, **dadurch gekennzeichnet, daß** als säuregruppenhaltiges Acrylat- oder (Meth)acrylat Polymeren (b) ein Copolymer aus 30 - 70 Gew.-% Ethylacrylat oder Methylmethacrylat und 70 - 30 Gew.-% Methacrylsäure enthalten ist.

7. Haft- und Bindemittel nach einem oder mehreren der Ansprüche 1 bis 4, **dadurch gekennzeichnet, daß** als säuregruppenhaltiges Acrylat- oder (Meth)acrylat Polymer (b) Polyacrylsäure enthalten ist.

8. Haft- und Bindemittel nach einem oder mehreren der Ansprüche 1 bis 7, **dadurch gekennzeichnet, daß** die kationischen Reste des kationischen (Meth)acrylat-Copolymers (a) mittels der Komponente (b) zu 2 - 100 % neutralisiert sind.

9. Haft- und Bindemittel nach einem oder mehreren der Ansprüche 1 bis 8, **dadurch gekennzeichnet, daß** als pharmazeutischer Wirkstoff ein Wirkstoff aus der therapeutische Kategorien der Analgetika, Antirheumatika, Antiallergika, Antiarrhythmika, Betarezeptorenblocker, Calciumkanalblocker, Hemmstoffe des Renin-Angiotensin-Systems, Broncholytika/Antiasthmatika, Cholinergika, Diuretika Durchblutungsfördernde Mittel, Gichtmittel, Grippemittel, Koronarmittel, Lipidsenker Magen-Darmmittel, Psychopharmaka, Thrombozytenaggregationshemmer, Urologika, Venetherapeutika, Vitamine oder Mineralien enthalten ist.

10. Haft- und Bindemittel nach Anspruch 9, **dadurch gekennzeichnet, daß** als pharmazeutischer Wirkstoff Morphin und/oder dessen Derivate,Tramadol, Acetylsalicylsäure, Diclofenac, Indometacin, Lonazolac, Ibuprofen, Ketoprofen, Propyphenazon, Naproxen, Paracetamol, Flurbiprofen, Dimetinden, Chinidin, Metoprolol, Propranolol, Oxprenolol, Pindolol, Atenolol, Metoprolol, Disopyramid, Verapamil, Diltiazem, Gallopamil, Nifedipin, Nicardipin, Nisoldipin, Nimodipin, Amlodipin, Theophyllin, Salbutamol, Terbutalin, Ambroxol, Aminophyllin, Cholintheophyllinat, Pyridostigmin, Piretanid, Furosemid, Pentoxifyllin, Naftidrofuryl, Buflomedil, Xantinolnicotinat, Bencyclan, Allopurinol, Norephedrin, Clorphenamin Isosorbidmononitrat, Isosorbiddinitrat, Glyceroltrinitrat, Molsidomin, Bezafibrat, Fenofibrat, Gemfibrozil, Cerivastatin, Pravastatin, Fluvastatin, Lovastatin, Atorvastatin, Simvastatin, Xantinol, Metoclopramid, Amitriptylin, Dibenzepin, Venlafaxin, Thioridazin, Oxazepam, Lithium, Nitrofurantoin, pflanzliche Trockenextrakt, Ascorbinsäure und Kalium und/oder deren pharmazeutisch verwendete Salze enthalten ist.

11. Haft- und Bindemittel nach Anspruch 9, **dadurch gekennzeichnet, daß** als pharmazeutischer Wirkstoff Nicotin, Glyceroltrinitrat, Scopolamin, Clonidin, Fentanyl, Östradiol, Testosteron, Oxibutynin, Diclophenac, Desoxyribonukleinsäuren z. B. für Vaccinen, Ibuprofen, Ketoprofen, Diltiazem, Propranolol, Albuterol, Alprazolam, Amethocaine, Atenolol, Benzoporphyrin, Buprenorphine, Calcitonin, Dithranol, Diphencypron, hautpenetrierende bzw. durch die Haut resorbierbare Peptide, Eptazocine, Ethinylöstradiol, Methrotrexat oder Naloxon enthalten ist.

12. Verfahren zur Herstellung eines Haft- und Bindemittel nach einem oder mehreren der Ansprüche 1 bis 11, indem man die Komponenten (a) bis (c) und gegebenenfalls (d) miteinander vermengt, mit oder ohne Zusatz von Wasser, durch Schmelzen, Spritzguß, Extrusion, Gießen, Ausstreichen, Aufsprühen oder Verpressen in das Überzugs- und Bindemittel überführt.

## Claims

1. Adhesive and binder for dermal or transdermal therapeutic systems, containing
a) a (meth)acrylate copolymer of radically polymerised C₁-C₄-alkyl esters of acrylic or methacrylic acid and (meth)acrylate monomers with a tertiary or quaternary amino group in the alkyl group, containing
b) 0.1 - 45 wt.%, based on (a), of an organic di- or tricarboxylic acid or an acid group-containing acrylate or (meth)acrylate polymer or copolymer, and
c) a substance which promotes penetration with regard to the skin,
d) optionally a pharmaceutical active substance, a plasticiser and/or one or more pharmaceutically conventional additives,
**characterised in that** the penetration-promoting substance (c) is an alcohol having 10 to 12 carbon atoms and at most 20 wt.% of a plasticiser are present, in relation to component (a).

2. Adhesive and binder according to claim 1, **characterised in that** the penetration-promoting substance (c) is decanol, undecanol or dodecanol.

3. Adhesive and binder according to claim 1 or 2, **characterised in that** the cationic (meth)acrylate copolymer (a) is made up of 30 to 80 wt.% of radically polymerised C₁-C₄-alkyl esters of acrylic or methacrylic acid and 70 to 20 wt.% of (meth)acrylate monomers with a tertiary amino group in the alkyl radical.

4. Adhesive and binder according to one or more of claims 1 to 3, **characterised in that** the cationic (meth)acrylate copolymer (a) is made up of 85 to 98 wt.% of radically polymerised C₁-C₄-alkyl esters of acrylic or methacrylic acid and 15 to 2 wt.% of (meth)acrylate monomers with a quaternary amino group in the alkyl radical.

5. Adhesive and binder according to one or more of claims 1 to 4, **characterised in that** succinic acid (succinate), fumaric acid or citric acid are present as the organic di- or tricarboxylic acids (b).

6. Adhesive and binder according to one or more of claims 1 to 4, **characterised in that** a copolymer of 30 - 70 wt.% of ethyl acrylate or methyl methacrylate and 70 - 30 wt.% of methacrylic acid is present as the acid group-containing acrylate or (meth)acrylate polymer (b).

7. Adhesive and binder according to one or more of claims 1 to 4, **characterised in that** polyacrylic acid is present as the acid group-containing acrylate or (meth)acrylate polymer (b).

8. Adhesive and binder according to one or more of claims 1 to 7, **characterised in that** the cationic groups of the cationic (meth)acrylate copolymer (a) are neutralised by 2 - 100% by means of component (b).

9. Adhesive and binder according to one or more of claims 1 to 7, **characterised in that** the pharmaceutical active substance present is an active substance selected from the therapeutic categories of the analgesics, antirheumatics, antiallergics, antiarrhythmics, beta-receptor blockers, calcium channel blockers, inhibitors of the renin-angiotensin system, broncholytics/antiasthmatics, cholinergics, diuretics, circulation promoters, anti-gout agents, anti-influenza agents, coronary drugs, lipid lowering agents, gastro-intestinal agents, psychoactive drugs, thrombocyte aggregation inhibitors, urological agents, vein therapy drugs, vitamins or minerals.

10. Adhesive and binder according to claim 9, **characterised in that** the pharmaceutical active substance present is morphine and/or the derivatives thereof, tramadol, acetylsalicylic acid, diclofenac, indomethacin, lonazolac, ibuprofen, ketoprofen, propyphenazone, naproxen, paracetamol, flurbiprofen, dimetinden, quinadine, metoprolol, propranolol, oxprenolol, pindolol, atenolol, metoprolol, disopyramide, verapamil, diltiazem, gallopamil, nifedipine, nicardipine, nisoldipine, nimodipine, amlodipine, theophylline, salbutamol, terbutaline, ambroxol, aminophylline, cholinetheophyllinate, pyridostigmine, piretanide, furosemide, pentoxifylline, naftidrofuryl, buflomedil, xantinol nicotinate, bencyclan, allopurinol, norephedrine, chlorphenamine isosorbide mononitrate, isosorbide dinitrate, glycerol trinitrate, molsidomine, bezafibrate, fenofibrate, gemfibrozil, cerivastatin, pravastatin, fluvastatin, lovastatin, atorvastatin, simvastatin, xantinol, metoclopramide, amitriptyline, dibenzepine, venlafaxine, thioridazine, oxazepam, lithium, nitrofurantoin, dry plant extract, ascorbic acid and potassium and the salts thereof which are used pharmaceutically.

11. Adhesive and binder according to claim 9, **characterised in that** the pharmaceutical active substance present is nicotine, glycerol trinitrate, scopolamine, clonidine, fentanyl, oestradiol, testosterone, oxibutynin, diclofenac, deoxyribonucleic acids, e.g. for vaccines, ibuprofen, ketoprofen, diltiazem, propranolol, albuterol, alprazolam, amethocaine, atenolol, benzoporphyrin, buprenorphone, calcitonin, dithranol, diphencypron, skin-penetrating or transdermally absorbed peptides, eptazocines, ethynyloestradiol, methotrexate or naloxone.

12. Method of manufacturing an adhesive and binder according to one or more of claims 1 to 11, wherein components (a) to (c) and optionally (d) are mixed together, with or without the addition of water, and converted into the binder by melting, injection moulding, extrusion, casting, spreading, spraying or compression.

## Revendications

1. Agent adhésif et liant destiné à des systèmes thérapeutiques dermiques ou trans-dermiques, contenant :
(a) un copolymère de (méth)acrylate d'esters d'alkyles en C₁ à C₄ de l'acide acrylique ou de l'acide méthacrylique polymérisés par voie radicalaire et de monomères de(méth)acrylate comportant un groupe amino tertiaire ou quaternaire dans le reste alkyle, contenant
(b) 0,1 - 45 % en poids, rapporté à (a) d'un acide organique di- ou tri-carboxylique ou d'un polymère ou d'un copolymère d'acrylate ou de (méth)acrylate contenant des groupes acides ainsi que
(c) une substance favorisant la pénétration par application sur la peau,
(d) le cas échéant, une substance active pharmaceutique, un plastifiant et/ou une ou plusieurs substance(s) additive(s) pharmaceutiquement courante(s),
**caractérisé en ce que**
la substance favorisant la pénétration (c) est un alcool comportant 10 à 12 atomes de carbone, et l'agent contient au maximum 20 % en poids d'un plastifiant, rapporté au composant (a).

2. Agent adhésif et liant selon la revendication 1,
**caractérisé en ce que**
la substance favorisant la pénétration (c) est le décanol, l'undécanol ou le dodécanol.

3. Agent adhésif et liant selon la revendication 1 ou 2,
**caractérisé en ce que**
le copolymère de (méth)acrylate cationique (a) est constitué de 30 à 80 % en poids d'esters d'alkyles en C₁ à C₄ de l'acide acrylique ou de l'acide méthacrylique polymérisés par voie radicalaire et de 70 à 20 % en poids de monomères de (méth)acrylate comportant un groupe amino tertiaire dans le reste alkyle.

4. Agent adhésif et liant selon une ou plusieurs des revendications 1 à 3,
**caractérisé en ce que**
le copolymère de (méth)acrylate cationique (a) est constitué de 85 à 98 % en poids d'esters d'alkyles en en C₁ à C₄ polymérisés par voie radicalaire de l'acide acrylique ou de l'acide méthacrylique polymérisés par voie radicalaire et de 15 à 2 % en poids de monomères de (méth)acrylate comportant un groupe amino quaternaire dans le reste alkyle.

5. Agent adhésif et liant selon une ou plusieurs des revendications 1 à 4,
**caractérisé en ce que**
comme acides organiques di- tricarboxyliques (b), il contient l'acide succinique (succinate), l'acide fumarique ou l'acide citrique.

6. Agent adhésif et liant selon une ou plusieurs des revendications 1 à 4,
**caractérisé en ce que**
comme polymère d'acrylate ou de (méth)acrylate comportant des groupes acides (b), il contient un copolymère de 30 - 70 % en poids d'acrylate d'éthyle ou de méthacrylate de méthyle et de 70 - 30 % en poids d'acide méthacrylique.

7. Agent adhésif et liant selon une ou plusieurs des revendications 1 à 4,
**caractérisé en ce que**
comme polymère d'acrylate ou de (méth)acrylate contenant des groupes acides (b), il contient l'acide polyacrylique.

8. Agent adhésif et liant selon une ou plusieurs des revendications 1 à 7,
**caractérisé en ce que**
les restes cationiques du copolymère de (méth)acrylate cationique (a) sont neutralisés au moyen du composant (b) à 2 - 100 %.

9. Agent adhésif et liant selon une ou plusieurs des revendications 1 à 8,
**caractérisé en ce que**
comme substance active pharmaceutique, il contient une substance active appartenant aux catégories thérapeutiques des analgésiques, antir-humatiques, anti-allergiques, anti-arythmiques, béta-bloquants, bloqueurs du canal du calcium, substances inhibitrices du système rénine-angiotensine, broncholytiques/anti-asthmatiques, cholinergiques, diurétiques, agents favorisant l'irrigation sanguine, agents de traitement de la goutte, agents anti- grippe, agents de traitement des affections coronariennes, agents de traitement pour l'estomac et l'intestin abaisseurs de lipides, psycho-pharmaceutiques, inhibiteurs d'agrégation de thrombocytes, urologiques, vénéthérapeutiques, vitamines ou minéraux.

10. Agent adhésif et liant selon la revendication 9,
**caractérisé en ce que**
comme substance(s) active(s) pharmaceutique(s), il contient la morphine et/ou ses dérivés, le tramadol, l'acide acétyl salicylique, le diclofenac, l'indométacine, le lonazolac, l'ibuprofène, le cétoprofène, la propyphénazone, le naproxène, le paracétamol, le flurbiprofène, le dimélindène, la quinidine, le métoprolol, le propandolol, l'oxprénolol, le pindolol, l'aténolol, le métoprolol, le disopyramide, le vérapamil, le diltiazeme ; le gallopamil, la nifédipine, la nicardipine, la nisoldipine, la nimodipine, l'amlodipine, la théophylline, le salbutamol, la terbutaline, l'ambroxol, l'aminophylline, le théophyllinate de choline, la pyridostigmine, le pirétanide, le furosémide, la pentoxyfilline, le naftidrofuryle, le buflomédil, le nicotinate de xantinol, le bencyclan, l'allopurinol, la noréphédrine, la chlorophénamine, le mononitrate d'isosorbide, le dinitrate d'isosorbide, le trinitrate de glycérol, la molsidomine, le bézafibrate, le fénofibrate, le gemfibrozil, la cérivastatine, la pravastatine, la fluvastatine, la lovastatine, l'atorvastatine, la simvastatine, le xantinol, le métoclopramide, l'amitriptyline, la dibenzépine, la venlafaxine, la thioridazine, l'oxazépame, le lithium, la nitrofurantoïne, un extrait sec végétal, l'acide ascorbique et le potassium et/ou leurs sels pharmaceutiquement utilisés.

11. Agent adhésif et liant selon la revendication 9,
**caractérisés en ce que**
comme substance pharmaceutique, il contient la nicotine, le trinitrate de glycérol, la scopolamine, la clonidine, le fentanyle, l'ostradiol, la testostérone, l'oxybutynine, le diclophénac, des acides désoxyribonucléïques par exemple pour les vaccins, l'ibuprofène, le cétoprofène, le diltiazème, le propanolol, l'albutérol, l'alprazolame, l'anéthocaïne, l'aténolol, la benzoporphyrine, la buprènorphine, la calcitonine, le dithranol, la diphencyprone, des peptides pénétrant par la peau ou résorbables par la peau, l'eptazocine, l'éthinylöstradiol, le méthrotrexat ou la naloxone.

12. Procédé pour la préparation d'un agent adhésif et liant selon une ou plusieurs des revendications 1 à 11, consistant à mélanger entre eux des composants (a) à (c) et, le cas échéant (d) avec ou sans addition d'eau, par fusion, moulage par injection, extrusion, coulée, enduction, pulvérisation ou compression, dans l'agent adhésif et liant.
